# EUROPÄISCHE PATENTSCHRIFT

(11) **EP 0 522 112 B1**
(45) Veröffentlichungstag und Bekanntmachung des Hinweises auf die Patenterteilung: **04.12.1996**
(21) Anmeldenummer: 92902606.0
(22) Anmeldetag: 03.01.1992
(51) Int. Cl.: C12P 7/18

(54) **VERFAHREN ZUR HERSTELLUNG VON DULCIT AUS LACTOSE**
METHOD OF PREPARING DULCITE FROM LACTOSE
PROCEDE POUR LA FABRICATION DE DULCITE A PARTIR DE LACTOSE

(30) Priorität: 24.01.1991 DE 4102243
(43) Veröffentlichungstag der Anmeldung: 13.01.1993
(73) Patentinhaber: SCHERING AKTIENGESELLSCHAFT, 13342 Berlin (DE)
(72) Erfinder: KLAGES, Uwe, D-1000 Berlin 28 (DE); WEBER, Alfred, D-1000 Berlin 37 (DE); KENNECKE, Mario, D-1000 Berlin 33 (DE)
(86) Internationale Anmeldenummer: DE9200008
(87) Internationale Veröffentlichungsnummer: WO9213085

(56) Entgegenhaltungen:
- JOURNAL OF BASIC MICROBILOGY Bd. 27, Nr. 9, 1987, Seiten 505 - 510; M. HELENE MARIN ET AL.: 'Strain Selection in Kluyveromyces marxianus var. lactis for galactose production' in der Anmeldung erwähnt
- JOURNAL OF BACTERIOLOGY Bd. 158, Nr. 2, Mai 1984, Seiten 705 - 712; MICHAEL I. RILEY ET AL.: 'Genetic and biochemical Characterization of the Galactose Gene Cluster in Kluyveromyces lactis' in der Anmeldung erwähnt

## Beschreibung

Die Erfindung betrifft ein Verfahren zur Herstellung von Dulcit durch fermentative Umsetzung von Lactose mittels galactokinase-negativen Mutanten der Gattung Kluyveromyces.

Es ist bekannt, daß galactokinase-negative Mutanten der Gattung Kluyveromyces die Fahigkeit besitzen das Disaccharid Lactose in Glucose und Galactose aufzuspalten, die Glucose zu metabolisieren und die Galactose nicht anzugreifen. (Michael I. Riley et. al. J. Bacteriol. 158, 1981, 705-712 und Pierre Galzy et. al., DE-A 24 34 874). Ferner ist bekannt, daß es auch solche Mutanten gibt, die in der Lage sind die bei dieser Umsetzung gebildete Galactose mit einer Ausbeute bis zu etwas 50 % zum Dulcit zu hydrieren (M.-Hélène Marin et. al. J. Basic Microbiol. 27, 1987, 505-510).

Will man auf diesem vorbekannten Wege Dulcit aus Lactose herstellen, so ist es erforderlich, diese zuerst mit einer galactokinase-negativen Mutante der Gattung Kluyveromyces umzuwandeln. die gebildete Galactose, oder das Gemisch aus Galactose und Dulcit zu isolieren und dann durch Hydrierung in Dulcit zu überführen (Beilstein 4. Auflage, 3. Erganzungswerk, Erster Band, 2405-2406. Dieses Verfahren ist aber wesentlich aufwendiger als die Gewinnung von Dulcit aus Madagaskar-Manna (Melampyrum nemorosum) (Römpps Chemie-Lexikon, 8. Auflage Franck'sche Verlagshandlung, Stuttgart, p 1027).

Das erfindungsgemäße Verfahren ermöglicht es, Lactose durch fermentative Umsetzung mit galactokinase-negativen Mutanten der Gattung Kluyveromyces in hohen Ausbeuten in Dulcit umzuwandeln. Das erhaltene Dulcit enthält praktisch keine oder nur geringe Mengen an Galactose (in aller Regel weniger als 5 %) und kann demzufolge durch einfache Umkristallisation - beispielsweise aus wässrigem Ethanol - aufgereinigt werden.

Das erfindungsgemäße Verfahren ist dadurch gekennzeichnet, daß man bei der fermentativen Umsetzung von Lactose mit galactokinase-negativen Mutanten der Gattung Kluyveromyces
a) zur Fermentationskultur nach weitgehender Hydrolyse der Lactose pro Liter 5 g bis 25 g Glucose hinzufügt oder
b) die Fermentation unter den Bedingungen des resting-cell Verfahrens durchführt.

Zur Durchführung des erfindungsgemäßen Verfahrens können die Mutanten von Kluyveromyces verwendet werden, welche in den bereits erwähnten Publikationen von Riley et. al., Galzy et. al. oder Marin et. al. beschrieben sind.

Da nicht alle Mutanten, die in diesen Publikationen erwähnt sind der Fachwelt frei zur Verfügung stehen, wurden unter den von Marin et. al. beschriebenen Bedingungen eigene Mutationsversuche durchgeführt und eine Mutante mit der internen Bezeichnung 144 EH erhalten, die in allen wesentlichen Merkmalen taxonomisch die Eigenschaften von Kluyveromyces marxianus var. lactis besitzt (J. Lodder "The Yeasts", North-Holland Publ. Comp., Amsterdam 1971, 349-352). Diese Mutante wurde bei der Deutschen Sammlung von Mikroorganismen und Zellkulturen GmbH, D-3300 Braunschweig unter der Nr. DSM 6119 hinterlegt, sie steht der Fachwelt frei zur Verfügung und erweist sicht als zur Durchführung des erfindungsgemäßen Verfahrens besonders geeignet.

Das erfindungsgemäße Verfahren wird unter den Bedingungen durchgeführt, die man auch bei den bekannnten mikrobiologischen Umwandlungen von Substraten mit Hefekulturen anwendet.

Unter den für Hefekulturen, insbesondere Kluyveromyceskulturen, üblicherweise verwendeten Kulturbedingungen werden in einem geeigneten Nährmedium unter Belüften und Rühren Vorkulturen angezüchtet.

Zur Durchführung der Verfahrensvariante a wird ein aliquoter Teil dieser Vorkultur in ein Fermentationsmedium überführt, welches neben den üblichen Nahrmedien pro Liter Kultur 10 g bis 50 g (insbesondere 20 g bis 30 g) Lactose enthält. Dann fermentiert man unter Rühren und Belüften bei einer Temperatur von 25° C bis 30° C und einem pH-Wert von 4 bis 6 bis etwa mindestens 95 % der Lactose hydrolytisch gespalten sind, setzt der Fermentationskultur pro g Lactose 0,25 g bis 1 g (vorzugsweise 0,4 g bis 0,6 g) Glucose zu und fermentiert weiter bis die Umsetzung zum Stillstand kommt.

Die optimale Lactosekonzentration und Glucosekonzentration, der optimale Zeitpunkt der Glucosezugabe und die optimale Fermentationsdauer sind von der Art der verwendeten Kluyveromyces Mutante und den Fermentationsbedingungen abhängig. Diese Größen müssen, wie dies bei mikrobiologischen Substratumwandlungen allgemein erforderlich ist im Einzelfall durch Vorversuche, wie sie dem Fachmann geläufig sind, ermittelt werden.

Zur Ermittlung des Fermentationsverlaufs kann man aus der Kultur in zeitlichen Abständen Proben entnehmen und in diesen in üblicher Weise - beispielsweise mittels Dunnschichtchromatographie - ihren Gehalt an Lactose, Galactose, Glucose und Dulcit ermitteln.

Zur Durchführung der Verfahrensvariante wird unter den Bedingungen der Variante a, aber ohne Zusatz von Lactose eine Kluyveromyces-Kultur angezüchtet. Nach einer Anwachsphase von etwa 24 bis 48 Stunden wird die Zellmasse durch Filtration oder Zentrifugieren abgetrennt, gewaschen und in einer gegebenenfalls isotonisierende Zusätze und Puffersubstanzen enthaltende 0,5 bis 10 (vorzugsweise 1 bis 5) gewichtsprozentige wässrige Lactoselösung so resuspendiert, daß die Zelldichte in dieser Suspension 2 bis 5 mal so groß ist, wie in der Fermentationskultur. Dann fermentiert man unter Rühren und Belüften bis die Umsetzung zum Stillstand kommt.

Auch in diesem Fall ist es erforderlich, die optimalen Reaktionsparameter durch Vorversuche zu ermitteln.

Nach erfolgter Umsetzung kann das Dulcit aus den gemäß Variante a oder b erhaltenen Fermentationsansatzen in einfacher Weise isoliert werden. Dies kann beispielsweise geschehen, indem man die Ansätze filtriert oder zentrifugiert, die erhaltenen Lösungen von anorganischen Salzen befreit, einengt und das Dulcit durch Zugabe geeigneter Lösungsmittel wie Ethanol zur Kristallisation bringt.

Das so dargestellte Dulcit kann - eventuell nach Umkristallisation - beispielsweise als zuckerfreier Süßstoff Anwendung finden.

Die nachfolgenden Ausführungsbeispiele dienen zur naheren Erlauterung des erfindungsgemäßen Verfahrens.

### Ausführungsbeispiele:

### Beispiel 1

Ein 2 l-Erlenmeyerkolben mit 1 l einer sterilen Nahrlosung enthaltend
5 g/l (NH₄)₂SO₄
1 g/l KH₂PO₄
0.5 g/l MgSO₄.7H₂O
0,1 g/l NaCl
0,1 g/l CaCl₂
400 µg/l Nicotinsaure
2 µg/l Biotin
400 µg/l Thiaminhydrochlorid
10 mg/l Adenin
5 g/l Lactose

- eingestellt auf pH 4 -
wird mit einer Impföse mit Zellen von Kluyveromyces marxianus DSM 6119 beimpft und 36 Stunden bei 30° C und 190 Umdrehungen pro Minute geschüttelt.

Ein 50 l-Fermenter mit 40 l einer sterilen Nahrlosung derselben Zusammensetzung wie die Vorkultur wird mit 1 l Vorkultur beimpft. Der Ansatz wird auf 30° C temperiert und 24 Stunden mit 100 Umdrehungen pro Minute gerührt und mit 1 m³ Luft pro Stunde belüftet.

Ein 50 l-fermenter mit 32 l einer sterilen Nährlösung derselben Zusammensetzung wie der Vorfermenter, jedoch mit 20 g/l Lactose und eingestellt auf pH 5, wird mit 4 l der Vorfermenter-Kultur beimpft und bei 30° C mit 100 Umdrehungen pro Minute gerührt und mit 1 m³ Luft pro Stunde belüftet.

Der pH-Wert wird so geregelt, daß er nicht unter pH 5 abfällt. Nach 48 Stunden wird eine sterile Lösung von 400 g Glucose in 4 l Wasser zugegeben.

Nach 96 Stunden wird der Ansatz mit je 100 g Aktivkohle und Perlite versetzt und filtriert. Die zellfreie Kulturbruhe wird über ein Elektrodialyse-Gerät auf einen Leitwert von 0,2 mS entsalzt. Die Losung wird im Vakuum auf etwa 1 l konzentriert und mit dem gleichen Volumen Ethanol versetzt. Als Kristallisat erhält man 374 g Dulcit.

### Beispiel 2

Ein 500 ml-Erlenmeyerkolben mit 100 ml einer sterilen Nährlosung enthaltend
3 g/l Hefeextrakt
3 g/l Malzextrakt
5 g/l Pepton
10 g/l Lactose
wird mit einer Impföse Zelle von Kluyveromyces marxianus DSM 6119 beimpft und 24 Stunden bei 30° C und 180 Umdrehungen pro Minute geschüttelt.

Die Zellen werden durch Zentrifugieren vom Medium getrennt, einmal mit 0,9 %iger Kochsalzlösung gewaschen und in 20 ml deionisiertem Wasser, enthaltend 0,5 g/l MgCl₂ und 10 g/l Lactose und eingestellt auf pH 4,5, resuspendiert.

Der Ansatz wird bei 30° C mit 180 Umdrehungen pro Minute geschüttelt. Nach 24 Stunden werden die Zellen abzentrifugiert. Durch DC-Analytik wird ermittelt, daß im Überstand 5 g/l Dulcit enthalten sind.

### Referenzbeispiel

Die Anzucht der Vorkultur und des Vorfermenters erfolgt wie in Beispiel 1 beschrieben.

Ein 50 l-Fermenter mit 36 l einer sterilen Nahrlosung derselben Zusammensetzung wie der Vorfermenter, jedoch mit 20 g/l Lactose und eingestellt auf pH 5, wird mit 4 l der Vorfermenter-Kultur beimpft und bei 30° C mit 100 Umdrehungen pro Minute gerührt und mit 1 m³ Luft pro Stunde belüftet. Der pH-Wert wird so geregelt, daß er nicht unter pH 5 abfallt.

Nach 72 Stunden wird der Ansatz mit je 100 g Aktivkohle und Perlite versetzt und filtriert. Die zellfreie Kulturbrühe wird über ein Elektrodialyse-Gerät auf einen Leitwert von 0,2 mS entsalzt. Die Lösung wird im Vakuum auf etwa 1 l konzentriert und mit dem gleichen Volumen Ethanol versetzt. Als Kristallisat erhält man 160 g Galactose und 212 g Dulcit.

## Patentansprüche

1. Verfahren zur Herstellung von Dulcit durch fermentative Umsetzung von Lactose mittels galactokinase-negativen Mutanten der Gattung Kluyveromyces, dadurch gekennzeichnet, daß man
a) zur Fermentationskultur nach weitgehender Hydrolyse der Lactose pro Liter 5 g bis 25 g Glucose hinzufügt oder
b) die Fermentation unter den 8edingungen des resting-cell Verfahrens durchführt.

2. Verfahren zur Herstellung von Dulcit gemäß Patentanspruch 1, dadurch gekennzeichnet, daß man zur Fermentation ein Kulturmedium verwendet, welches pro Liter 10 g bis 50 g Lactose enthält.

3. Verfahren zur Herstellung von Dulcit gemäß Patentanspruch 1, dadurch gekennzeichnet, daß man die Fermentation mittels der Mutante Kluyveromyces marxianus DSM 6119 durchführt.

## Claims

1. Process for the preparation of dulcite by fermentative reaction of lactose using galactokinase-negative mutants of the Kluyveromyces genus, characterised in that
a) after extensive hydrolysis of the lactose, from 5 g to 25 g of glucose are added per litre to the fermentation culture, or
b) the fermentation is carried out under the conditions of the resting-cell process.

2. Process for the preparation of dulcite according to patent claim 1, characterised in that there is used for the fermentation a culture medium that contains from 10 g to 50 g of lactose per litre.

3. Process for the preparation of dulcite according to patent claim 1, characterised in that the fermentation is carried out using the mutant Kluyveromyces marxianus DSM 6119.

## Revendications

1. Procédé de préparation de dulcitol par transformation fermentative de lactose à l'aide de mutants de *Kluyveromyces* négatifs pour la galactokinase, caractérisé en ce que
a) on ajoute au milieu de culture de fermentation, après une hydrolyse poussée du lactose, 5 g à 25 g de glucose par litre, ou
b) on effectue la fermentation dans les conditions du procédé des cellules en période de repos.

2. Procédé de préparation de dulcitol selon la revendication 1, caractérisé en ce que l'on utilise pour la fermentation un milieu de culture qui contient 10 g à 50 g de lactose par litre.

3. Procédé de préparation de dulcitol selon la revendication 1, caractérisé en ce que l'on effectue la fermentation à l'aide du mutant *Kluyveromyces marxianus* DSM 6119.
